# EUROPEAN PATENT SPECIFICATION

(11) **EP 1 360 550 B1**
(45) Date of publication and mention of the grant of the patent: **03.01.2007**
(21) Application number: 02711603.7
(22) Date of filing: 12.02.2002
(51) Int. Cl.: G03B 42/02, A61B 6/06

(54) **COLLIMATOR ARRANGEMENT**
KOLLIMATORANORDNUNG
SYSTEME COLLIMATEUR

(30) Priority: 12.02.2001 SE 0100470; 12.02.2001 US 268156 P
(43) Date of publication of application: 12.11.2003
(73) Proprietor: Sectra Mamea AB, 164 40 Kista (SE); Arcoma AB, 352 46 Växjö (SE)
(72) Inventor: DANIELSSON, Mats, S-187 43 Täby (SE); LAMMROTH, Theresa, S-125 44 älvsjö (SE); RAPP, Lennart, S-214 25 Malmö (SE)
(74) Representative: Dahnér, Christer
(86) International application number: PCT/SE2002/000238
(87) International publication number: WO 2002/065209

(56) References cited:
- EP-A1- 0 426 285
- EP-A1- 1 120 086
- DE-A1- 19 939 678
- US-A- 4 203 037
- US-A- 4 542 521
- US-A- 4 930 143
- US-A- 5 627 869

## Description

### TECHNICAL FIELD OF THE INVENTION

The present invention enables scanned-slit x-ray imaging in mammography in the sense that for the first time a high spatial resolution image can be registered without compromising the ergonomics in positioning the breast. It further relates to a beam collimator arrangement consisting of one or several collimators in an x-ray apparatus shaping the x-ray beam to hit the active detector area (pre-collimator) and to remove scattered x-rays (aft collimator). It further comprises: an x-ray source; an x-ray image receiver positioned to receive x-rays from the x-ray source; means for positioning the object to be examined, the means being positionable between the x-ray source and the x-ray image receiver; and said pre collimator positioned between the x-ray source and the means for positioning the object.

### BACKGROUND OF THE INVENTION

When carrying out scanned-slit mammography, one essential problem is placing the breast of a patient in right position while at the same time positioning the moving collimator is as close as possible to the breast to obtain a high image quality in terms of spatial resolution.

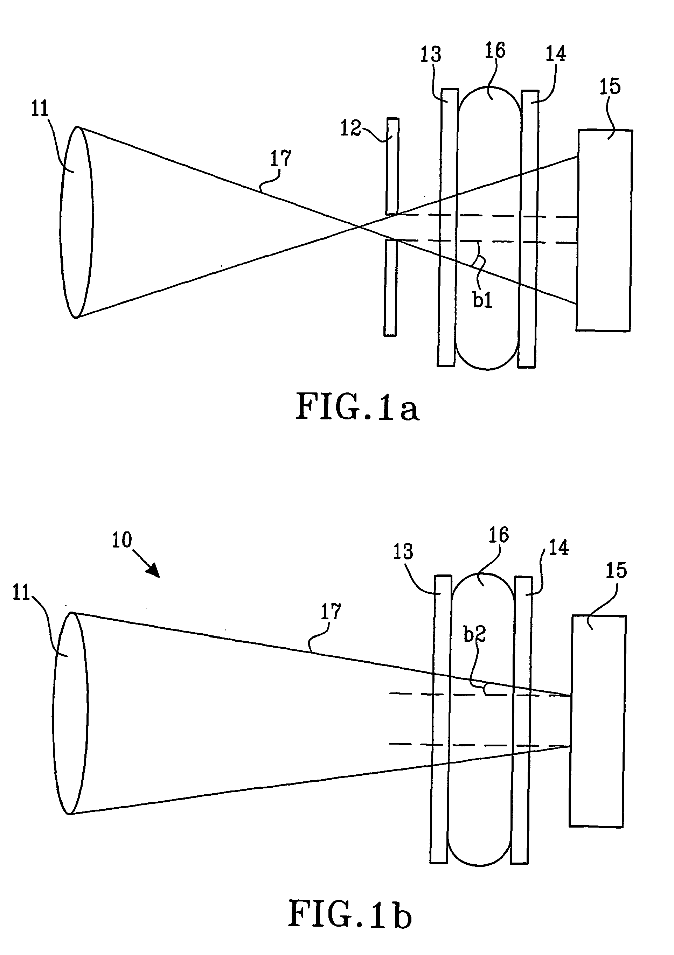

Fig. 1 a is a schematic side view of a digital mammography x-ray equipment 10 comprising an x-ray source 11, a collimator arrangement 12, compression paddles 13 and 14, and a detector assembly 15. X-rays are denoted with 17.

The breast 16 to be examined (x-rayed) is placed between the compression paddles 13 and 14 and exposed to a pressure, which prevents motion during the exposure and also decreases the amount of overlapping tissue since the breast is flattened out.

As illustrated in Figs. 1a and 1b, the collimator arrangement 12 must be placed as close as possible to the upper compression paddle 12. As indicated in Fig. 1a the image quality will be compromised if this is not the case, due to size of the x-ray source that will be imaged on the detector through the slit the spatial resolution bl will be deteriorated if the collimator is moved away from the object. This is only true in one dimension, the dimension orthogonal to the slit, as indicated in Fig. 1b. Thus, when placing the breast in the examining position, one must avoid the collimator arrangement, and when the examining starts the collimator must be placed as close as possible to and above the upper paddle.

EP 0417 965 discloses a method and apparatus for improving imaging in an X-ray mammography machine having a controllable X-ray source incorporates apparatus for directing the X-ray beam in a preselected configuration onto at least a portion of a target area. The creation intensity of the beam is detected in each of a plurality of substantially equal segments of the target area and a first segment of lowest radiation intensity is identified. The X-ray source is adjusted to establish a predetermined radiation intensity in the identified first segment. Second segments are then identified each having a radiation intensity greater than a predetermined percentage of the radiation intensity in the identified first segment. The radiation intensity directed toward the identified second segments is then automatically attenuated by a control system responsive to the radiation intensity detected in the second segments. The attenuation is achieved by moving collimators into the beam so as to reduce the radiation directed towards the second segments. The beam is scanned over the target and continuously adjusted in accordance with the radiation detected in each of the segments of the beam. In this manner, each area of the target receives just sufficient radiation to provide proper imaging without overexposing area of the target, which have less attenuation than other areas. Thus, the object of this invention is to obtain attenuation by horizontally displacing the collimator.

A mammography apparatus for obtaining mammography images with optimal beam collimation is disclosed in US 5,627,869. Proportional control of collimator element position based upon compression paddle position and dimension inputs is provided to obtain optimal collimation of the X-ray beam for a desired imaging technique and compressed breast thickness. Relevant objects of this invention are: to provide a mammography apparatus with an X-ray beam collimator that is automatically adjusted to provide an appropriately collimated X-ray beam for any magnification factor, and to provide a mammography apparatus with an X-ray beam collimator that is automatically adjusted to provide an X-ray beam that is appropriately collimated for any magnification factor for a breast of any thickness using a compression paddle of any dimension. Hence, the collimator is provided with collimator blades, which are controlled to be displaced in horizontal direction.

US 4,203,037discloses an apparatus for exposing an object to x-ray radiation including adjustable object supporting and compressing means and first collimator means having an opening and being disposed between the x-ray generator and the object supporting means.
Second collimator means having an opening and being disposed between the obj ect supporting means and x-ray detecting means. Means are provided for moving the first and second collimator means in such fashion as to maintain alignment between the openings therein and the x-ray generator, in order to sequentially expose portions of the object of x-ray passing through the first collimator means and permit such x-ray to pass through the opening in a second collimator means and impinge upon the x-ray detecting means. The object supporting and compressing means is preferably structurally independent of the collimator means. The x-ray detecting means preferably takes the form of a highly sensitive detector such as a film-screen cassette or a self-scanning array of photodiodes optically coupled to scintillator means.

### SUMMARY OF THE INVENTION

Thus, the main object of the present invention is to provide a collimator arrangement and an x-ray apparatus employing such a collimator arrangement that solves the problems associated with the prior art and allow a working field for the nurse or other personal that is free from obstacles. Another object of the present invention is to provide a collimator arrangement that can be placed as closes as possible to the object to be examined during the exposure.

Therefore the initially mentioned collimator arrangement is arranged on a carrying structure to displace the beam collimator arrangement between a first position when no x-ray exposure is conducted and a second position right before x-ray exposure is initiated and during the x-ray exposure. Preferably, the second position is in a substantially short distance from said compressing means. For flexibility, said displacement is in lateral and or horizontal direction.

The invention also relates to a mammography apparatus as defined in claim 1 and a method in a mammography apparatus as defined in claim 4.

### BRIEF DESCRIPTION OF THE DRAWINGS

In the following, the invention will be further described in a non-limiting way with reference to the accompanying drawings in which:
- Figs. 1a;1b: are schematic illustrations of an x-ray apparatus according to priori art,
- Fig 2: illustrates in a schematic way an embodiment of an x-ray apparatus according to the present invention, and
- Fig 3: illustrates in a schematic way a more detailed embodiment of an x-ray apparatus according to the present invention.

### DETAILED DESCRIPTION OF THE EMBODIMENTS

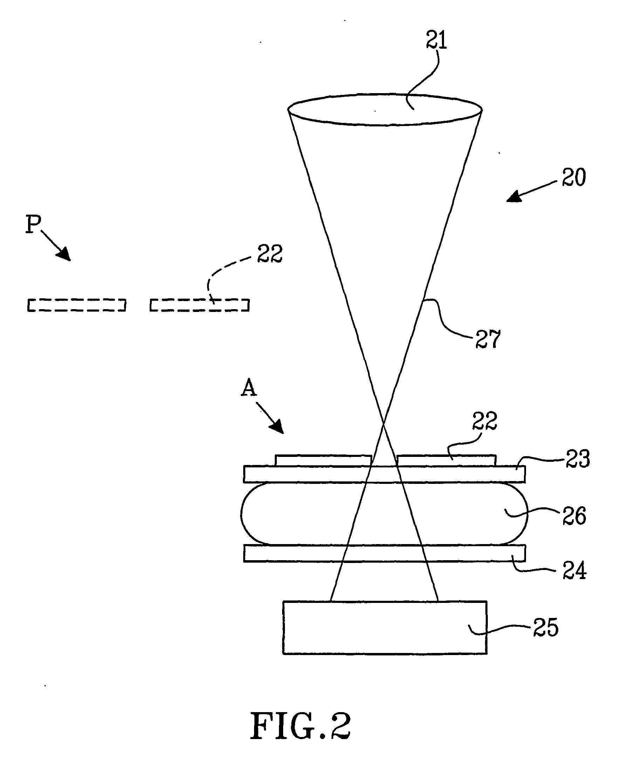

Fig. 2 is a schematic view, illustrating an x-ray apparatus 20 according to the present invention. The apparatus 20 comprises an x-ray source 21; a collimator arrangement 22, compression paddles 23 and 24, and a detector assembly 25. The breast 26 to be examined (x-rayed) is placed between the compression paddles 23 and 24 and exposed to a pressure, which flattens it so that as much as the breast is exposed to the x-rays and detected by the detector assembly 25 and a sufficient contrast is obtained. The x-rays are denoted with 27.

The collimator arrangement 22 is arranged to assume at least two positions, a parking position P and an active position A. In the parking position the collimator is arranged in an area, which does not interfere with the positioning of the patient's breast between the compression paddles. This area can be an area outside the exposure area, preferably, by displacing and/or lifting the collimator arrangement from the exposure area. Displacement of the entire collimator arrangement is a lateral movement while the lifting is a vertical movement with respect to the plane of the drawing.

When the examination starts, the collimator arrangement 22 is placed in the parking position P. It must be high or offset enough to not disturb the nurse's adjustment of the breast 26. When the compression is completed and the exposure to x-ray is to be initiated, the collimator arrangement 22 moves downwards/sideway and stops in a position as close as possible to the compression plate 23. When the exposure is completed the collimator arrangement moves upwards/sideways with at least the speed of the compression plate to the parking position. The collimator motion towards the compression paddles can be initiated, e.g. by a dedicated switch after the positioning of the breast is carried out. It is important that the time the breast needs to be compressed is minimized. For this reason the movement of the collimator arrangement should be as fast as possible without causing patient anxiety. An exact parking position for the collimator is possible to configure, through commands of a control system, between approximately 18 cm from detector and approximately 50 cm from the detector In particular, when multi-slits collimators are used, it is important that the collimator is placed close to the object to be examined. In this case, the resolution of the resultant image is deteriorated in a direction perpendicular to the collimator's slits.

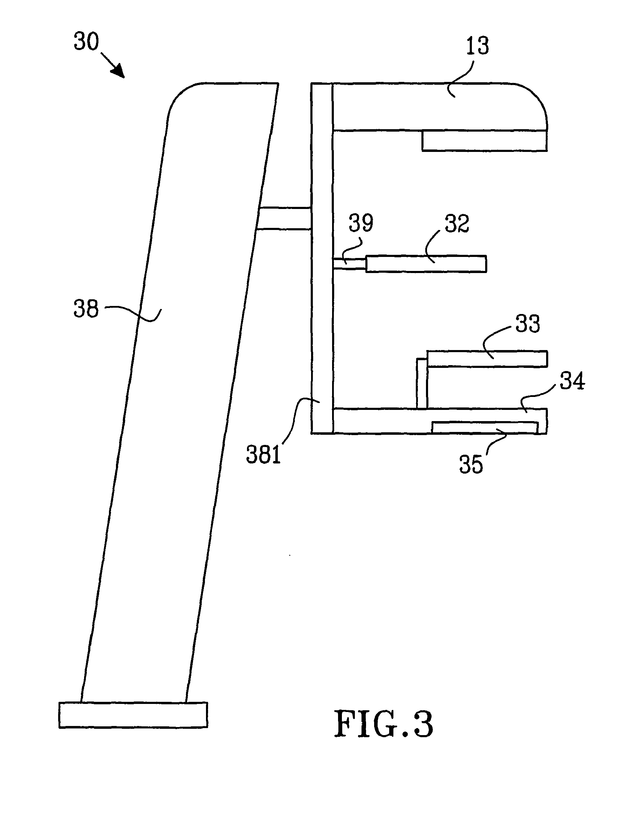

Fig. 3 is a schematic, but more detailed illustration, showing an x-ray apparatus 30 according to the invention. The apparatus 30 generally comprises a base 38, an imaging arm 381 pivotally attached to the base 38, an x-ray source 31 fixed to one end of the imaging arm 381, an image receiver or detector assembly 35 fixed at a second end of the imaging arm. The apparatus also includes compression paddle holders, which are slidably attached to the imaging arm. The compression paddle holder holds upper and lower compression paddles 33 and 34, respectively. A displaceable collimator arrangement 32 is arranged on a collimator arm 39. According to this embodiment, the detector assembly 35 is integrated with the lower compression paddle 34.

The collimator holder arm 39 is arranged to displace the collimator arrangement 32 by sliding the collimator in a vertical direction and also it can comprise a telescopic structure to displace the collimator arrangement horizontally. In Fig. 3, the collimator arrangement 32 is situated in its parking position.

The invention is not limited to mammography and can be used in any type of x-ray imaging apparatuses, using semiconducting or gaseous detectors or x-ray film.

The invention is not limited to the shown embodiments; it can be varied in a number of ways without departing from the scope of the appended claims.

## Claims

1. A mammography apparatus (20, 30) comprising:
o an X-ray source (21, 31);
o an X-ray image receiver (25, 35) positioned to receive X-rays from the X-ray source;
o first and second means (23, 33; 24; 34) for compressing tissue, said means being arranged to be positioned between the X-ray source and the X-ray image receiver, said means further having a compression surface of predetermined dimension;
o a beam collimator (22, 32) positioned between the X-ray source and said means for compressing tissue;
**characterized in**
**that** said apparatus further comprises means (39) for displacing said beam collimator (22, 32) between a first position (P) outside the exposure area when no X-ray exposure is conducted and a second position (A) near the tissue before X-ray exposure is initiated and that said first position is vertically and horizontally displaced with respect to said second position.

2. The apparatus of claim 1,
**characterised in**
**that** said second position is in a substantially short distance from said compressing means (23, 33).

3. The apparatus of claim 1,
**characterised in that** the first position (P) is located vertically above the second position (A).

4. A method in a mammography apparatus (20, 30), said apparatus comprising:
o an X-ray source (21,31);
o an X-ray image receiver (25, 35) positioned to receive X-rays from the X-ray source;
o first and second means (23, 33; 24; 34) for compressing tissue, said means being arranged to be positioned between the X-ray source and the X-ray image receiver, said means further having a compression surface of predetermined dimension;
o a beam collimator (22, 32) positioned between the X-ray source and said means for compressing tissue;
o means (39) for displacing said beam collimator (22, 32)
the method comprising the steps of:
placing said means (39) in a first position (P) outside the exposure area when no X-ray exposure is conducted and
placing said means (39) a second position (A) near the tissue before X-ray exposure is initiated, wherein said first position is vertically and horizontally displaced with respect to said second position.

## Patentansprüche

1. Mammographievorrichtung (20, 30), umfassend:
- eine Röntgenquelle (21, 31);
- einen Röntgenstrahlbildempfänger (25, 35), welcher positioniert ist, um Röntgenstrahlen von der Röntgenquelle zu empfangen;
- erste und zweite Mittel (23, 33; 24; 34) zum Zusammendrücken von Gewebe, wobei die Mittel eingerichtet sind, um zwischen der Röntgenquelle und dem Röntgenstrahlbildempfänger positioniert zu werden, wobei die Mittel weiterhin eine Druckfläche mit einer vorherbestimmten Abmessung aufweisen;
- einen Strahlkollimator (22, 32), welcher zwischen der Röntgenquelle und den Mitteln zum Zusammendrücken von Gewebe positioniert ist;
**dadurch gekennzeichnet,**
**dass** die Vorrichtung weiterhin Mittel (39) zum Versetzen des Strahlkollimators (22, 32) zwischen einer ersten Position (P) außerhalb des Belichtungsbereichs, wenn keine Röntgenbelichtung ausgeführt wird, und einer zweiten Position (A) nahe dem Gewebe, bevor eine Röntgenbelichtung eingeleitet wird, umfasst, und dass die erste Position vertikal und horizontal bezüglich der zweiten Position versetzt ist.

2. Vorrichtung nach Anspruch 1,
**dadurch gekennzeichnet,**
**dass** sich die zweite Position in einer im Wesentlichen kurzen Entfernung von den Zusammendrückmitteln (23, 33) befindet.

3. Vorrichtung nach Anspruch 1,
**dadurch gekennzeichnet,**
**dass** die erste Position (P) vertikal oberhalb der zweiten Position (A) angeordnet ist.

4. Verfahren bei einer Mammographievorrichtung (20, 30),
wobei die Vorrichtung umfasst:
- eine Röntgenquelle (21, 31);
- einen Röntgenstrahlbildempfänger (25, 35), welcher positioniert ist, um Röntgenstrahlen von der Röntgenquelle zu empfangen;
- erste und zweite Mittel (23, 33; 24; 34) zum Zusammendrücken von Gewebe, wobei die Mittel eingerichtet sind, um zwischen der Röntgenquelle und dem Röntgenstrahlbildempfänger positioniert zu werden, wobei die Mittel weiterhin eine Druckfläche mit einer vorherbestimmten Abmessung aufweisen;
- einen Strahlkollimator (22, 32), welcher zwischen der Röntgenquelle und den Mitteln zum Zusammendrücken von Gewebe positioniert ist;
- Mittel (39) zum Versetzen des Strahlkollimators (22, 32)
wobei das Verfahren die Schritte umfasst:
Platzieren der Mittel (39) in einer ersten Position (P) außerhalb des Belichtungsbereich, wenn keine Röntgenbelichtung ausgeführt wird, und Platzieren der Mittel (39) in einer zweiten Position (A) nahe dem Gewebe bevor eine Röntgenbelichtung eingeleitet wird, wobei die erste Position vertikal und horizontal bezüglich der zweiten Position versetzt ist.

## Revendications

1. Appareil de mammographie (20, 30) comportant :
- une source de rayons X (21, 31),
- un récepteur d'image de rayons X (25, 35) positionné pour recevoir des rayons X provenant de la source de rayons X,
- des premiers et seconds moyens (23, 24 ; 24 ; 34) pour comprimer un tissu, lesdits moyens étant conçus pour être positionnés entre la source de rayons X et le récepteur d'image de rayons X, lesdits moyens ayant de plus une surface de compression présentant une dimension prédéterminée,
- un collimateur de faisceau (22, 32) positionné entre la source de rayons X et lesdits moyens pour comprimer un tissu,
**caractérisé en ce que**
ledit appareil comporte de plus des moyens (39) pour déplacer ledit collimateur de faisceau (22, 32) entre une première position (P) à l'extérieur de la zone d'exposition où aucune exposition de rayons X n'est effectuée et une seconde position (A) à proximité du tissu avant que l'exposition de rayons X soit initiée et **en ce que** ladite première position est déplacée verticalement et horizontalement par rapport à ladite seconde position.

2. Appareil selon la revendication 1,
**caractérisé en ce que**
ladite seconde position est à une distance essentiellement courte desdits moyens de compression (23, 33).

3. Appareil selon la revendication 1,
**caractérisé en ce que** la première position (P) est située verticalement au-dessus de la seconde position (A).

4. Procédé dans un appareil de mammographie (20, 30), ledit appareil comportant :
- une source de rayons X (21, 31),
- un récepteur d'image de rayons X (25, 35) positionné pour recevoir des rayons X provenant de la source de rayons X,
- des premiers et seconds moyens (23, 33 ; 24 ; 34) pour comprimer un tissu, lesdits moyens étant conçus pour être positionnés entre la source de rayons X et le récepteur d'image de rayons X, lesdits moyens ayant de plus une surface de compression présentant une dimension prédéterminée,
- un collimateur de faisceau (22, 32) positionné entre la source de rayons X et lesdits moyens pour comprimer un tissu,
- des moyens (39) pour déplacer ledit collimateur de faisceau (22, 32)
le procédé comportant les étapes consistant à :
placer lesdits moyens (39) à une première position (P) à l'extérieur de la zone d'exposition où aucune exposition de rayons X n'est effectuée et placer lesdits moyens (39) à une seconde position (A) à proximité du tissu avant que l'exposition de rayons X soit initiée, ladite première position étant déplacée verticalement et horizontalement par rapport à ladite seconde position.
